# EUROPÄISCHE PATENTSCHRIFT

(11) **EP 0 663 922 B1**
(45) Veröffentlichungstag und Bekanntmachung des Hinweises auf die Patenterteilung: **03.11.1999**
(21) Anmeldenummer: 94924305.9
(22) Anmeldetag: 30.07.1994
(51) Int. Cl.: C07H 19/10, C07H 19/20, C07F 9/572, C12Q 1/68, C07H 21/00

(54) **INFRAROT-FARBSTOFF-MARKIERTE NUCLEOTIDE UND IHRE VERWENDUNG IN DER NUCLEINSÄURE-DETEKTION**
INFRA-RED DYE-LABELLED NUCLEOTIDES AND THEIR USE IN NUCLEIC ACID DETECTION
NUCLEOTIDES MARQUES AU COLORANT A L'INFRAROUGE ET LEUR UTILISATION POUR LA DETECTION D'ACIDES NUCLEIQUES

(30) Priorität: 06.08.1993 DE 4326466
(43) Veröffentlichungstag der Anmeldung: 26.07.1995
(73) Patentinhaber: Roche Diagnostics GmbH, 68298 Mannheim (DE)
(72) Erfinder: MUEHLEGGER, Klaus, D-82398 Polling (DE); HOELTKE, Hans-Joachim, D-82327 Tutzing (DE); BIRKNER, Christian, D-82449 Uffing (DE); VON DER ELTZ, Herbert, D-82362 Weilheim (DE); LYLE Middendorf, Lincoln, Nebraska 68505 (US)
(86) Internationale Anmeldenummer: EP9402541
(87) Internationale Veröffentlichungsnummer: WO9504747

(56) Entgegenhaltungen:
- EP-A- 0 359 225
- EP-A- 0 527 433
- WO-A-90/03383
- JOURNAL OF ORGANIC CHEMISTRY OF THE USSR. (ZHURNAL ORGANICHESKOI KHIMII), Bd.57, 1992, NEW YORK US Seiten 4578 - 4580 STREKOWSKI ET AL 'Substitution Reactions of a Nucleofugal Group in Heptamethine Cyanin Dyes. Synthesis of an Isocyanato Derivative for Labeling of Proteins with a Near-Infrared Chromophore'

## Beschreibung

Die Erfindung betrifft Nucleosid-5'-triphosphate und Phosphoramidite, die am Basenteil bzw. am Phosphoratom einen im Langwelligen absorbierenden fluoreszierenden Rest, vorzugsweise eine Carbocyanin-Gruppe tragen, sowie deren Verwendung zur Markierung, Detektion und Sequenzierung von Nucleinsäuren.

Nucleinsäuren haben als Träger bzw. Überträger der genetischen Information eine zentrale Bedeutung in der belebten Natur. Sie haben deshalb seit ihrer Entdeckung durch F. Miescher das breite Interesse der Naturwissenschaften erregt und zur Aufklärung ihrer Funktion, Struktur und Wirkungsweise geführt. Mit der zunehmenden Kenntnis dieser grundlegenden molekularbiologischen Mechanismen ist es in den letzten Jahren möglich geworden, die Neukombination von Genen zu betreiben. Diese Technologie eröffnet z.B. neue Möglichkeiten in der medizinischen Diagnose und Therapie und in der Pflanzenzüchtung.

Ein wesentliches Werkzeug zur Erklärung dieser Zusammenhänge und der Lösung der Probleme war und ist der Nachweis der Nucleinsäuren und zwar sowohl was ihren spezifischen Nachweis betrifft, als auch was ihre Sequenz, also ihre Primärstruktur angeht.

**Die spezifische Nachweisbarkeit** von Nucleinsäuren beruht auf der Eigenschaft dieser Moleküle, mit anderen Nucleinsäuren durch Ausbildung von Basenpaarungen über Wasserstoffbrücken in Wechselwirkung zu treten, zu "hybridisieren". In geeigneter Weise markierte, d. h. mit Indikatorgruppen versehene Nucleinsäuren ("Sonden") können so zum Nachweis komplementärer Nucleinsäuren ("target") eingesetzt werden.

Die **Ermittlung der Primärstruktur** ("Sequenz"), also der Abfolge der heterocyclischen Basen einer Nucleinsäure geschieht mittels den Techniken der "Sequenzierung". Diese Kenntnis der Sequenz ist wiederum die Grundvoraussetzung für einen gezielten und spezifischen Einsatz von Nucleinsäuren in molekularbiologischen Fragestellungen und Arbeitstechniken. Auch die Sequenzierung bedient sich letztlich des Prinzips der spezifischen Hybridisierung von Nucleinsäuren untereinander. Dabei werden wie oben erwähnt ebenfalls markierte Nucleinsäure-Fragmente verwendet.

Aus dem Gesagten wird deutlich, daß die geeignete Markierung von Nucleinsäuren eine unverzichtbare Voraussetzung jeglicher Nachweismethode ist.

Schon frühzeitig wurde dafür vor allem die radioaktive Markierung mit geeigneten Isotopen, wie ³²P oder ³⁵S eingesetzt. Die Nachteile der Verwendung radioaktiver Reagentien liegen jedoch klar auf der Hand: entsprechende Arbeiten bedürfen spezieller räumlicher Einrichtungen und Genehmigungen, sowie einer kontrollierten und aufwendigen Entsorgung des radioaktiven Abfalls. Die Reagentien zur radioaktiven Markierung sind teuer. Eine längere zeitliche Aufbewahrung derart markierter Proben ist wegen der kurzen Halbwertszeit obiger Nuklide nicht möglich.

Es hat daher in den letzten Jahren nicht an Versuchen gefehlt, diese gravierenden Nachteile zu umgehen, d. h. von einer radioaktiven Markierung wegzukommen. Dabei sollte die hohe Sensitivität dieser Markierungsart möglichst beibehalten werden.
Hier sind in der Tat bereits große Fortschritte erzielt worden [siehe z. B "Nonradioactive Labeling and Detection of Biomolecules", C. Kessler (Hrsg.) Springer Verlag Berlin, Heidelberg 1992].

Als nicht-radioaktive Indikatormoleküle haben sich u. a. hauptsächlich Haptene (wie Biotin oder Digoxigenin), Enzyme (wie alkalische Phosphatase oder Peroxidase) oder Fluoreszenzfarbstoffe (wie Fluorescein oder Rhodamin) bewährt.

Obwohl die **Markierung mit Haptenen** wie z. B. Digoxigenin in den Empfindlichkeitsbereich der Radioaktivität reicht, ist ein der radioaktiven Markierung entsprechender direkter Nachweis Hapten-markierter Nucleinsäuren nicht möglich. Hier muß eine Detektionsreaktion nachgeschaltet werden, die beispielsweise über eine Antikörper-Reaktion erfolgt. Diese indirekte Detektion erfordert mehrere Schritte, d. h. mehr Zeit und finanziellen Aufwand. Da Proteine zur Nachweisreaktion eingesetzt werden, ist eine spezielle Behandlung der Festphase (Membranen, Mikrotiterplatten) durch Blockieren und Waschschritte nötig, um eine unspezifische Bindung zu reduzieren. Dennoch ist die Sensitivität dieser Zweistufen-Detektion in der Regel durch Entstehen von störender Hintergrundfärbung aufgrund unspezifischer Proteinbindung limitiert. Für direkt enzymmarkierte Nucleinsäuren gilt prinzipiell dasselbe.

Den genannten Nachteil der oben geschilderten indirekten Detektion weist die Verwendung von Fluoreszenz-markierten Nucleinsäuren nicht auf. Ein direkter Nachweis durch Anregung der Fluoreszenz ist im Prinzip möglich und mit geeigneter Einrichtung (Fluoreszenz-Mikroskop, Scanner) sicht- und messbar. Auch hier stört jedoch die Autofluoreszenz von Zell- und Gewebekomponenten des zu untersuchenden biologischen Materials, wie Farbstoffe, Lipide, Proteine etc. Solche Störungen treten insbesondere auch bei Einsatz von festen Trägermaterialien (z.B. Nylon-Membranen) durch deren Eigenfluoreszenz auf und erschweren oder verhindern den Nachweis.

Eine Lösung dieser Probleme bietet sich prinzipiell durch die Verwendung von Farbstoffen an, deren Excitation und Emmission in Wellenlängenbereichen über 680 nm, also im nahen Infrarot(NIR)-Bereich liegt.
Hier fallen die o. g. störenden Einflüsse nicht mehr ins Gewicht. Ein weiterer wesentlicher Vorteil besteht darin, daß zur Anregung billige Laserdioden mit hoher Lebensdauer verwendet werden können.

So ist z.B. die Technik der DNA-Sequenzierung nach Fluoreszenz-Markierung der DNA-Fragmente durch photoelektrische Messung mit einem Laser und einem Sensor Gegenstand einer Anmeldung (US 4,729,947). Dabei werden in an sich bekannter Weise nach dem sogenannten Sanger-Verfahren IR-Farbstoff-markierte Oligonucleotide als Primer eingesetzt, die dabei als Starter der Synthese des neuen, komplementaren Nucleinsäurestranges fungieren.
Diese Methode hat aber den Nachteil, daß-abhängig von der zu sequenzierenden DNA-jeweils spezifische, d. h. eine Vielzahl von solchen markierten Primern immer wieder neu zu synthetisieren sind. Auch ist diese Synthese markierter oligomerer Primer mit hohem zeitlichem und finanziellem Aufwand verbunden, da erst das unmarkierte Oligonucleotid hergestellt werden muß und anschließend die Signal(Reporter)gruppe in einer zweiten Reaktion chemisch angebracht wird.

Es bestand daher die Aufgabe, Verbindungen herzustellen, die eine universelle, einfache und spezifische Markierung von Nucleinsäuren ermöglichen.
Nun ist bekannt, daß sich Nucleinsäuren durch den Einbau von entsprechend markierten Nucleosid-triphosphaten mit Polymerasen neu synthetisieren und damit markieren lassen. Auf dem Sektor der Desoxyribonucleinsäuren (DNA) geschieht dies durch DNA-Polymerasen nach den Methoden der "nick translation" [Rigby, P. W. et al. (1977) J. Mol. Biol. 113, 237] und des "random primed labeling" [Feinberg, A. P. & Vogelstein, B. (1984) Anal. Biochem. **137**, 266] durch den Einbau von Desoxy-nucleotiden, im Falle der Ribonucleinsäuren durch RNA-Polymerasen und Ribonucleotiden im Sinne einer Transcription. Eine weitere Methode der Markierung von Nucleinsäuren ist über eine sogenannte "3'-tailing"-Reaktion mit Hilfe von Terminaler Transferase und Ribo- bzw. Desoxyribonucleosid-triphosphaten möglich.

Mit Indikatormolekülen versehene Nucleosid-triphosphate wie Fluorescein oder Digoxigenin (MW 332 bzw. 390 ) werden jedoch -im Vergleich zu ihren natürlichen Substraten - von Polymerasen nur noch relativ schlecht als Substrate akzeptiert und in die neusynthetisierte Nucleinsäure eingebaut (Hoeltke, H.-J. et al (1990) Biol. Chem. Hoppe-Seyler **371**, 929).

Es war daher nicht zu erwarten, daß Indikatormoleküle mit noch deutlich höheren Molekulargewichten (800-1000) von Polymerasen als Substrate angenommen und in Nucleinsäuren eingebaut werden würden. Es war noch weniger zu erwarten, daß diese Moleküle mit der gegebenen räumlich anspruchsvollen Struktur infolge starker sterischer Hinderung von Polymerasen umgesetzt werden würden.

Überraschend wurde nun gefunden, daß mit Infrarot-Farbstoffen markierte Nucleosidtriphosphate von Polymerasen wie T7 DNA Polymerase als Substrate akzeptiert und in Nucleinsäuren eingebaut werden. Die erfindungsgemäßen Verbindungen sind daher neu.

Eine weitere Aufgabe der Erfindung bestand darin, eine Methode der Verwendung der oben genannten erfindungsgemäßen markierten Nucleotide zu finden, die es erlaubt, die damit markierten Nucleinsäuren direkt auf festen Trägern wie z. B. Nylon-Membranen oder in Lösung, z. B. in Mikrotiterplatten zu detektieren.
Wie oben bereits beschrieben, hat die Markierung von Nucleinsäuren mit Fluorophoren wie Fluorescein oder Tetramethylrhodamin den Nachteil, daß die Messung dieser Fluoreszenz durch die Eigenfluoreszenz des Trägermaterials gestört wird.
Werden nun aber die erfindungsgemäßen IR-Farbstoff-markierten Nucleosid-triphosphate zur Markierung der Nucleinsäuren verwendet, so fallen diese Störungen wegen der im nahen Infrarot-Bereich gelegenen Messwellenlängen nicht mehr ins Gewicht.

Die Vorteile einer Nucleinsäure-Detektion durch *in situ* Hybridisierung sind bekannt. Gewöhnlich werden dabei die markierten Proben oder Sonden entweder direkt unter dem Fluoreszenzmikroskop detektiert oder aber im Falle der Hapten-Markierung über einen weiteren Verfahrensschritt in einer immunologischen Reaktion ("ELISA") nachgewiesen. Diese Sichtbarmachung geschieht in der Regel durch Fixierung der Proben an z. B. Nylonmembranen oder in flüssiger, homogener Phase in Mikrotiterplatten. Dieser zusätzliche Schritt ist mit höherem zeitlichem und finanziellem Aufwand verbunden. Es ist also wünschenswert, diesen Schritt wegfallen zu lassen.

Durch die Möglichkeit der direkten Anregung der IR-Fluoreszenz-markierten Nucleinsäuren durch geeignete Laserdioden und die oben geschilderte Unempfindlichkeit der Detektion gegen Eigenfluoreszenz des Trägermaterials ist eine einfache und kostengünstige apparative Ausstattung möglich. Die immunologische Nachweisreaktion kann entfallen. Der Nachweis der IR-markierten Nucleinsäure geschieht einfach optisch durch die Kombination Laser/Detektor mit Hilfe eines geeigneten Scanners oder Mikrotiterplatten -Readers.

Zusammenfassend kann gesagt werden, daß die Verwendung der erfindungsgemäßen mit Infrarot-Fluorophoren markierten Nucleosid-5'-triphosphate als Polymerase-Substrate den direkten enzymatischen Einbau in Nucleinsäuren ermöglichen und eine Detektion der so markierten Nucleinsäuren sowohl für Sequenzierungen, als auch durch eine in dieser Kombination ebenfalls neue und daher ebenfalls erfindungsgemäße *in situ* Hybridisierung zulassen.

Die erfindungsgemäßen Nucleosid-5'-triphosphate der allgemeinen Formel 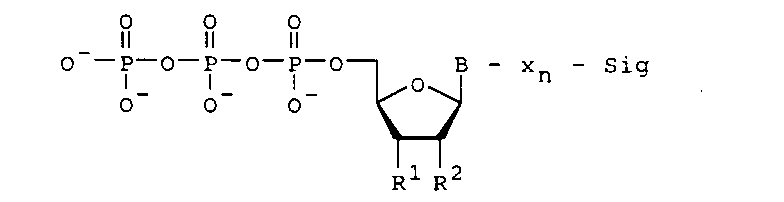 werden hergestellt, indem in an sich bekannter Weise von unmodifizierten Nucleosiden ausgegangen wird, diese im Falle der Pyrimidinnucleoside Uridin, Thymidin und Cytidin bzw. der Purin-nucleoside Adenosin und Guanosin, sowie der diesen entsprechenden 7-Desaza-purin- und 7-Desaza-8-aza-purin-nucleoside in geeigneter Weise an C-5 oder C-6 (Pyrimidine), an C-8 (Purine), an C-8 (3-Desaza-purine) an C-7 oder C-8 (7-Desaza-purine) chemisch modifiziert und letztlich 5'-phosphoryliert.

Die modifizierende Gruppe besteht zweckmäßigerweise aus einem Abstandhalter geeigneter Länge (spacer), sowie einer terminalen primären oder sekundären Aminogruppe, die durch geeignete aktivierte Fluoreszenzfarbstoffe (z. B. in Form der Isothiocyanate oder N-Hydroxysuccinimidester) substituierbar ist.

Soche Fluoreszenzfarbstoffe werden in aktiverter, d.h. mit z. B. Aminogruppen gut reagierender Form eingesetzt, vorzugsweise als Isothiocyanate. Nach erfolgter Reaktion sind die Fluorophore über NHCS-Gruppen an die modifizierende Gruppe des Nucleotides kovalent gebunden.

Die Phosphorylierung der so modifizierten Nucleoside erfolgt nach in der Literatur bekannten Verfahren [z. B. Yoshikawa, M. et al. (1967) Tetrah. Lett. **50**, 5065] durch Umsetzung mit Phosphoroxidtrichlorid zum Monophosphat und anschließender Reaktion mit Pyrophosphorsäure zum gewünschten 5'-Triphosphat.
Alternativ ist auch eine direkte Modifizierung der präformierten Nucleosid-5'-triphosphate möglich.

Die Fluorophore sind Verbindungen, die im nahen Infrarot-Bereich absorbieren, d. h. zwischen 600 und 800 nm. Bevorzugt sind solche von 630 nm bis 780 nm, wie z. B. Carbocyanine.

Wie schon oben erwähnt ist für die Zwecke der Sequenzierung neben der oben geschilderten Methode des Einbaus von markierten Nucleosid-triphospaten mit Polymerasen auch ein weiteres Verfahren üblich, das sich der Verwendung von markierten Oligonucleotiden, sogenannten Primern bedient. Wie ebenfalls erwähnt, ist die übliche Synthese dieser Moleküle in einer mehrstufigen Reaktion sehr zeitaufwendig. Die Signalgruppe muß dabei nach abgeschlossener Oligonucleotidsynthese in einem Extra-Schritt am 5'-Ende des Oligomeren angebracht werden. Da die eigentliche Oligonucleotidsynthese in automatisch arbeitenden Synthesegeräten erfolgt, ist es in hohem Maße wünschenswert, daß der Schritt des Anbringens der Signalgruppe ebenfalls bereits im Synthesizer erfolgen kann. Da die genannte Oligonucleotid-Synthese in einem schrittweisen Anhängen von monomeren Bausteinen, sogenannten Nucleosid-phosphoramiditen besteht, war es eine weitere Aufgabe der Erfindung, ein Fluorophor-phosphoramidit zu entwickeln, welches den direkten Einbau der Signalgruppe als letzten Schritt in der automatischen Oligonucleotidsynthese ermöglicht. Ein solches NIR-Farbstoff-phosphoramidit ist bislang nicht bekannt und daher erfinderisch neu.

Die folgenden Beispiele erläutern die Erfindung näher.

### Beispiel 1

### 5-(3-Aminoallyl)-2'-desoxy-uridin -5'-triphosphat

Dieses Derivat wurde wie von Langer et al. in Proc. Natl. Acad. Sci. USA (1981) **78**, 6635 beschrieben hergestellt.

### Beispiel 2

### Anhydro-11-phenoxy-10,12-propylen-3,3,3',3'-tetramethyl-4,5-benzindoindotricarbocyanin-1-(4-sulfobutyl)-1'-(3-aminopropyl)-thiono-[5-(3-aminoallyl)-2'-desoxy-uridin-5'-triphosphat]

Zu einer Lösung von 33 mg 5-Aminoallyl-dUTP-Li₄ (60 µmol) in 2 ml 0,1 m Naboratpuffer, pH 8,5 , wird eine Lösung von 50 mg Anhydro-11-phenoxy-10,12-propylen-3,3,3',3'-tetramethyl-4,5-benzindo-1-(4-sulfobutyl)-1'-(3-isothiocyanopropyl)-indotricarbocyanin-Na-Salz (60 µmol) in 1 ml Dimethylformamid gegeben und das Reaktionsgemisch ca. 15 Stunden lichtgeschützt bei Raumtemperatur stehen gelassen. Danach ist laut Papierelektrophorese (0,05 m Na-Citrat-Puffer, pH 5) der größte Teil der Ausgangsmaterialien umgesetzt. Zur Isolierung der gewünschten Substanz wird das Reaktionsgemisch mit ca. 50 ml Wasser verdünnt und die tiefgrün gefärbte Lösung auf eine Ionenaustauschersäule mit DEAE-Sephadex A-25 in der Chloridform gegeben. Das Produkt wird mit einem linearen Gradient von Wasser auf 1m LiCI von der Säule eluiert, die Produktfraktionen im Vakuum konzentriert und über eine "Reversed Phase"-Chromatographie an RP 18-Material entsalzt. Nach Lyophilisation erhält man 6 µmol (10 %) des gewünschten Triphosphates.

Spektrale Daten: Emissionₘₐₓ 786 nm, 720 nm (Schulter), 238 nm

### Beispiel 3:

### Anhydro-10,12-propylen-3,3,3',3'-tetramethyl-1,1'-bis(3-sulfobutyl)-indotricarbocyanin-11-(4-amino)phenoxy-thiono-[8-(5-aminopentylamino)-2'-desoxyadenosin-5'-triphosphat] ("IRD-dATP")

Das Derivat wird nach dem unter Beispiel 2 angegebenen Verfahren aus 38 mg 8-Aminopentylamino-dATP (60 µmol) und 50 mg Anhydro-10,12-propylen-3,3,3',3'-tetramethyl-1,1'-bis(3-sulfobutyl)-11-(4-isothiocyano)phenoxy-indotricarbocyanin-Na-Salz (60 µmol) hergestellt. Es wurden 3 µmol der Verbindung erhalten.

Spektrale Daten: Emission Max. 770 nm, 697 nm (Schulter), 279 nm

### Beispiel 4:

### Einsatz von IRD-dATP als Substrat für T7-DNA-Polymerase

3 µg Template DNA werden in einem Gemisch von 2 µl Reaktionspuffer (200 mM Tris-HCl, pH 7,5 , 100 mM MgCl₂, 250 mM NaCl), 1 pM M13/pUC-Primer und 7 µl H₂O 15 Min. bei 37 °C inkubiert.
Für die Markierungsreaktion werden 1 µl DTT (100 mM), 2 µl Markierungsmix (10 µM IRD 40-dATP, je 1 µM dCTP, dGTP und d TTP), 1 µl H₂O und 2 µl T7-DNA-Polymerase (2,5 U/µl) zugegebenund bei RT 10 Min. inkubiert.

Zur Verwendung in der DNA-Sequenzierung erfolgt anschließend eine Terminationsreaktion durch Zugabe der Terminationsmixe (ddATP, ddGTP, ddCTP, ddTTP).

### Beispiel 5:

### Anhydro-11-phenoxy-10,12-propylen-3,3,3',3'-tetramethyl-4,5-benzindoindotricarbocyanin-1-(4-sulfobutyl)-1'-(3-aminopropyl)-thiono-[5-(3-aminoallyl)-uridin-5'-triphosphat]

Die Verbindung wurde analog Beispiel 2 aus 5-Aminoallyl-UTP (nach Beispiel 1 hergestellt) und dem entsprechenden Isothiocyanat synthetisiert.

Die spektralen Daten entsprechen der 2'-Desoxy-Verbindung aus Beispiel 2.

### Beispiel 6:

### Anhydro-10,12-propylen-3,3,3',3'-tetramethyl-1,1'-bis(3-sulfobutyl)-indotricarbocyanin-11-(4-amino)phenoxy-thiono-[5-(3-aminoallyl)-2',3'-didesoxy-uridin-5'-triphosphat] ("IRD -ddUTP")

### 1.Stufe: 2',3'-didesoxy-uridin-5'-triphosphat

Das Derivat wurde ausgehend von kommerziell erhältlichem 2',3'-Didesoxy-cytidin-5'-triphosphat (Boehringer Mannheim) durch Desaminierung mit NaNO₂/Essigsäure über das instabile Diazonium-Derivat synthetisiert.

### 2. Stufe: 5-(3-aminoallyl)-2',3'-didesoxy-uridin-5'-triphosphat

Die Verbindung wurde analog Beispiel 1 nach Langer et al. über das 5-Mercuri-Derivat des 2',3'-didesoxy-UTP hergestellt.

### 3.Stufe: "IRD-ddUTP"

Das Didesoxy-Derivat wurde entsprechend Beispiel 2 durch Umsetzung des 5-Aminoallyl-ddUTP mit dem entsprechenden Isothiocyanat erhalten

Die spektralen Daten entsprechen denen der 2'-Desoxy-Verbindung aus Beispiel 3

### Beispiel 7:

### Anhydro-10,12-propylen-3,3,3',3'-tetramethyl-1,1'-bis(3-sulfopropyl)-indotricarbocyanin-11-[(4-ethoxy)phenoxy-O-(2-cyanoethyl)-N,N-diisopropylphosphoramidit

In einem 50 ml Rundkolben werden 425 mg Anhydro-11-(4-hydroxyethyl)phenoxy-10,12-propylen-3,3,3',3'-tetramethyl-1,1'-bis (3-sulfopropyl)-indotricarbocyanin-hydroxid in Form des Na-Salzes (0,5 mmol) in 5 ml trockenem Acetonitril gelöst und dazu 0,275 ml Ethyldiisopropylamin (1,6 mmol) gegeben. Anschließend tropft man unter Stickstoff und Rühren 0,125 ml Chlor-β-cyanoethoxy-N,N-diisopropylamino-phosphan innerhalb von ca. 3 Min. ein. Man rührt weitere 30 Min. bei RT, fügt dann ca. 10 ml wässrige, 5%ige NaHCO₃-Lösung zu und extrahiert daraufhin 2x mit je ca. 10 ml Dichlormethan. Die vereinigten organischen Phasen werden über Na-Sulfat getrocknet, das Lösungsmittel abdestilliert und der Rückstand an Kieselgel mit dem Laufmittel Dichlormethan/Ethylacetat/Triethylamin 45:45:10 chromatographiert.
Die Ausbeute beträgt 480 mg = 88,7 % d. Th.

DC (Kieselgel, Fließmittel wie o. a. ) R_{f}= 0,4
³¹P-NMR (d₆DMSO): 149 und 153 ppm (2 Diastereomere)

## Patentansprüche

1. Nucleosid-5'-triphosphate der allgemeinen Formel 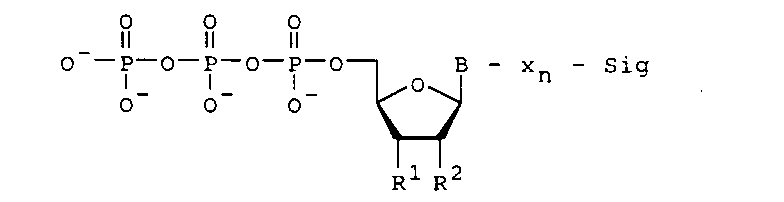 worin
B die heterocyclischen Basen Adenin, Guanin, Hypoxanthin, 7-Desaza-adenin, 7-Desaza-guanin, 7-Desaza-hypoxanthin, 7-Desaza-8-aza-adenin, 7-Desaza-8-aza-guanin, 8-Desaza-8-aza-hypoxanthin sowie Thymin, Cytosin und Uracil bedeutet, x eine verbindende Gruppe mit n= 4-20 Atomen,
Sig ein Carbocyanin der Anregungswellenlänge 650-800 nm
und R¹ und R² jeweils H und/oder OH darstellen, und
wobei diese Nukleosid-5'-triphosphate als Substrate von Polymerasen akzeptiert und in Nukleinsäuren eingebaut werden.

2. Verbindungen nach Anspruch 1, worin B die oben angegebene Bedeutung hat, x eine verbindende Gruppe mit vorzugsweise n=10-15 Atomen darstellt,
Sig ein Carbocyanin der allgemeinen Formel 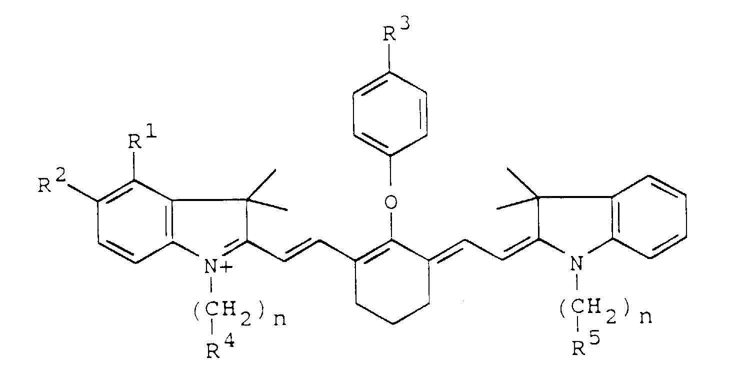 darstellt, worin R₁ und R₂ = H oder zusammen einen Phenylrest,
R₃ = H im Falle der Verknüpfung mit dem Nucleotid über die R₄ Position, bzw. -NHCS-im Falle der Verknüpfung über die R₃ Position,
R₄ und R₅ jeweils Sulfonyl und n = 3-5 oder R₄ = -NHCS- mit n = 3-8,
R₅ = Sulfonyl und n = 3-5 im Falle der Verknüpfung über die R₄ Position bedeuten.

3. Verwendung der Verbindungen nach den Ansprüchen 1 oder als Substrate für DNA-Polymerasen in einer Probe.

4. Verwendung der Verbindungen nach den Ansprüchen 1 oder 2 als Substrate für RNA-Polymerasen in einer Probe.

5. Verwendung der Verbindungen nach den Ansprüchen 1 oder 2 zur Markierung von Nucleinsäuren in einer Probe.

6. Verwendung der Verbindungen nach den Ansprüchen 1 oder 2 zum Nachweis von Nucleinsäuren in einer Probe.

7. Verwendung der Verbindungen nach den Ansprüchen 1 oder 2 in der DNA-Sequenzierung.

8. Verwendung der Verbindungen nach den Ansprüchen 1 oder 2, indem diese in der *in situ*-Hybridisierung eingesetzt werden.

9. Verwendung der Verbindungen nach den Ansprüchen 1-2, dadurch gekennzeichnet, daß die Hybridisierung auf Membranen erfolgt.

10. Verwendung der Verbindungen nach den Ansprüchen 1-2, dadurch gekennzeichnet, daß die Hybridisierung in Lösung durchgeführt wird.

11. Verwendung der Verbindungen nach den Ansprüchen 1-2, dadurch gekennzeichnet, daß die Hybridisierung in Lösung in Mikrotiterplatten durchgeführt wird.

12. Verwendung der Verbindungen nach Anspruch 6, dadurch gekennzeichnet, daß die Detektion der markierten Hybride mittels entsprechender Laserdioden und Detektoren erfolgt.

13. Verbindungen der allgemeinen Formel 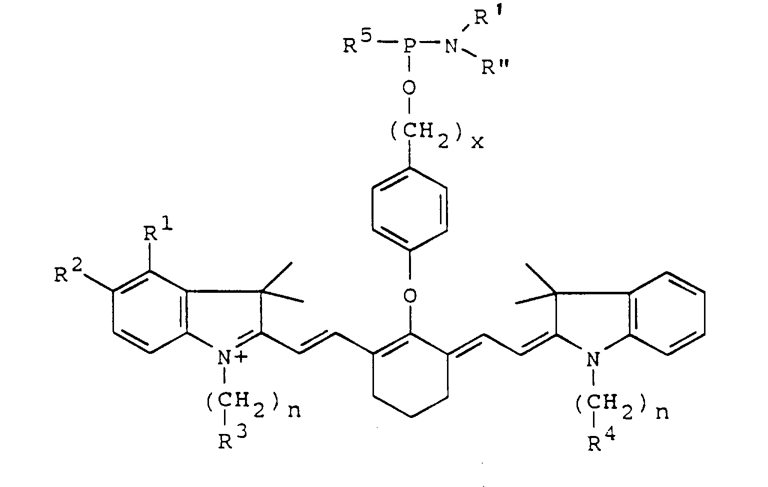 worin
R₁ und R₂ = H oder zusammen einen Phenylrest,
R₃ und R₄ jeweils Sulfonyl mit n = 3-5, R₅ = Methoxy oder 2-Cyanethoxy und R^{I} und R^{II} jeweils Ethyl oder Isopropyl und x = 1-10 bedeuten.

14. Verwendung der Verbindungen nach Anspruch 13, dadurch gekennzeichnet, daß diese in der Oligonucleotidsynthese nach dem Phosphoramidit-Verfahren eingesetzt werden.

15. Verwendung der Verbindungen nach Anspruch 14 zur 5'-Markierung von Oligonucleotiden.

## Claims

1. Nucleoside-5'-triphosphates of the general formula 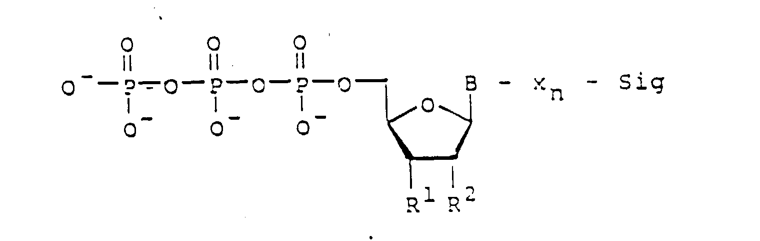 in which
B denotes the heterocyclic bases adenine, guanine, hypoxanthine, 7-deaza-adenine, 7-deaza-guanine, 7-deaza-hypoxanthine, 7-deaza-8-aza-adenine, 7-deaza-8-aza-guanine, 8-deaza-8-aza-hypoxanthine as well as thymine, cytosine and uracil,
x represents a linking group in which n = 4-20 atoms,
Sig represents a carbocyanine with an excitation wavelength 650-800 nm
and R¹ and R² each represent H and/or OH and
wherein these nucleoside-5'-triphosphates are accepted as substrates by polymerases and are incorporated into nucleic acids.

2. Compounds as claimed in claim 1, in which B has the meaning stated above, x represents a linking group with preferably n = 10 -15 atoms,
Sig represents a carbocyanine of the general formula 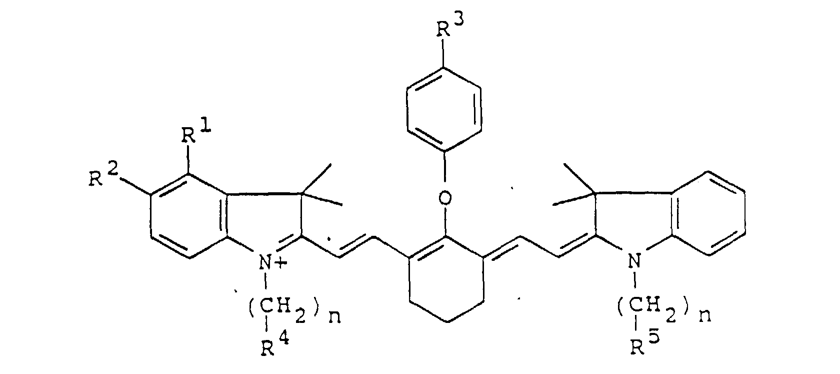 in which R₁ and R₂ = H or together represent a phenyl residue,
R₃ = H if linkage with the nucleotide is via the R₄ position or NHCS if linkage is via the R₃ position,
R₄ and R₅ each denote sulfonyl and n = 3-5 or R₄ = -NHCS with n = 3-8,
R₅ denotes sulfonyl and n = 3-5 if linkage is via the R₄ position.

3. Use of the compounds as claimed in claims 1 or 2 as substrates for DNA polymerases in a sample.

4. Use of compounds as claimed in claims 1 or 2 as substrates for RNA polymerases in a sample.

5. Use of compounds as claimed in claims 1 or 2 to label nucleic acids in a sample.

6. Use of compounds as claimed in claims 1 or 2 to detect nucleic acids in a sample.

7. Use of compounds as claimed in claims 1 or 2 in DNA sequencing.

8. Use of compounds as claimed in claims 1 or 2, by using these in *in situ* hybridization.

9. Use of compounds as claimed in claims 1 to 2, wherein the hybridization is carried out on membranes.

10. Use of compounds as claimed in claims 1 to 2, wherein the hybridization is carried out in solution.

11. Use of compounds as claimed in claims 1 to 2, wherein the hybridization is carried out in solution in microtitre plates.

12. Use of compounds as claimed in claim 6, wherein the labelled hybrids are detected by means of appropriate laser diodes and detectors.

13. Compounds of the general formula 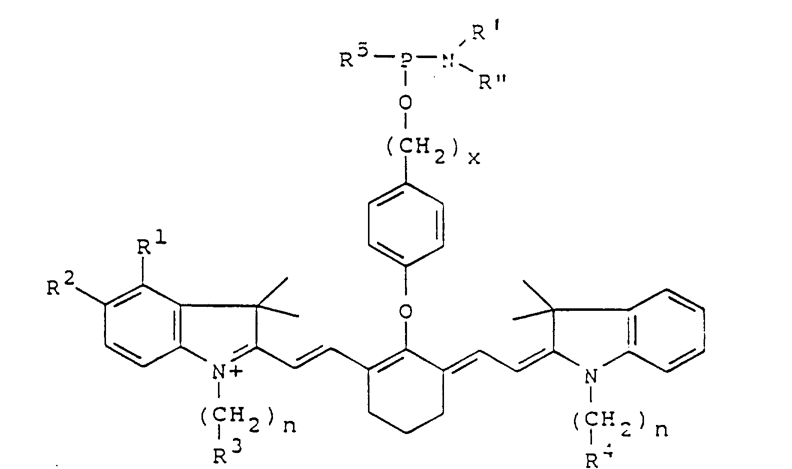 in which
R₁ and R₂ = H or together denote a phenyl residue,
R₃ and R₄ each denote sulfonyl with n = 3-5, R₅ = methoxy or 2-cyanoethoxy and R^{I} and R^{II} each denotes ethyl or isopropyl and X = 1-10.

14. Use of compounds as claimed in claim 13, wherein these are used for oligonucleotide synthesis by the phosphoramidite process.

15. Use of compounds as claimed in claim 14 to 5'-label oligonucleotides.

## Revendications

1. Nucléoside-5'-triphosphate de formule générale 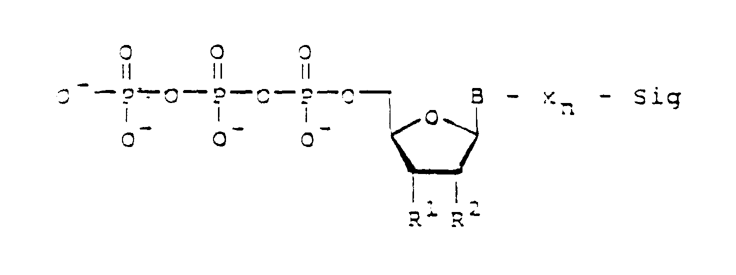 dans laquelle
B représente les bases hétérocycliques adénine, guanine, hypoxanthine, 7-désaza-adénine, 7-désaza-guanine, 7-désaza-hypoxanthine, 7-désaza-8-aza-adénine, 7-désaza-8-aza-guanine, 8-désaza-8-azahypoxanthine ainsi que la thymine, la cytosine et l'uracil, x étant un groupe de liaison avec n = 4-20 atomes,
Sig représente une carbocyanine dont la longueur d'onde d'excitation est comprise entre 650 et 800 nm, et R¹ et R² represent chacun H et/ou OH ces nucléoside-5'-triphosphates sont acceptés comme substrats de polymérases et incorporés dans des acides nucléiques.

2. Composés selon la revendication 1, dans lesquels B a la signification donnée plus haut, x représente un groupe de liaison avec n = 10-15 atomes,
Sig représente une carbocyanine de formule générale 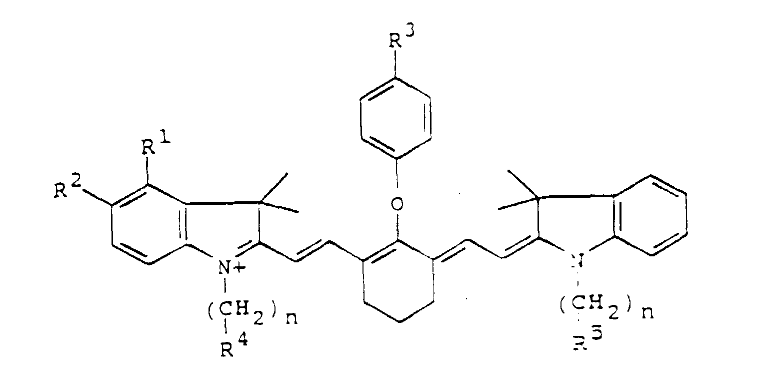 dans laquelle R₁ et R₂ = H ou forment ensemble un groupe phényle,
R₃ = H en cas de liaison au nucléotide par l'intermédiaire de la position R₄, ou - NHCS- en cas de liaison par l'intermédiaire de la position R₃,
R₄ et R₅ représentent chacun un sulfonyle et n = 3-5 ou R₄ = -NHCS- avec n = 3-8,
R₅ = sulfonyle et n = 3-5 en cas de liaison par l'intermédiaire de la position R₄.

3. Utilisation des composés selon les revendications 1 ou 2 comme substrats de polymérases d'ADN dans un échantillon.

4. Utilisation des composés selon les revendications 1 ou 2 comme substrats de polymérases d'ARN dans un échantillon.

5. Utilisation des composés selon les revendications 1 ou 2 pour le marquage d'acides nucléiques dans un échantillon.

6. Utilisation des composés selon les revendications 1 ou 2 pour la détection d'acides nucléiques dans un échantillon.

7. Utilisation des composés selon les revendications 1 ou 2 dans le séquençage d'ADN.

8. Utilisation des composés selon les revendications 1 ou 2 dans laquelle ceux-ci sont utilisés en hybridation *in situ.*

9. Utilisation des composés selon les revendications 1 - 2, caractérisée en ce que l'hybridation s'effectue sur des membranes.

10. Utilisation des composés selon les revendications 1 - 2, caractérisée en ce que l'hybridation s'effectue en solution.

11. Utilisation des composés selon les revendications 1 - 2, caractérisée en ce que l'hybridation s'effectue en solution dans des plaquettes de microtitration.

12. Utilisation des composés selon la revendication 6, caractérisée en ce que la détection des hybrides marqués s'effectue au moyen de diodes laser et de détecteurs appropriés.

13. Composés de formule générale 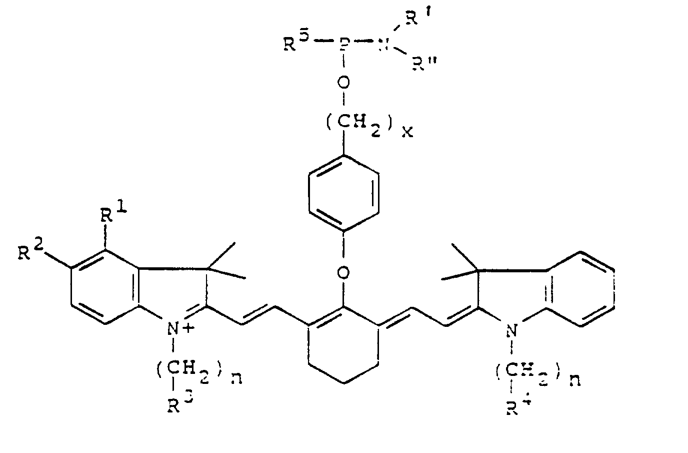 dans laquelle
R₁ et R₂ = H ou forment ensemble un groupe phényle, R₃ et R₄ représentent chacun un sulfonyle avec n = 3-5, R₅ = méthoxy ou 2-cyanoéthoxy et R' et R" représentent chacun un éthyle ou un isopropyle, et x = 1-10.

14. Utilisation des composés selon la revendication 13, caractérisée en ce que ceux-ci sont utilisés en synthèse d'oligonucléotides par le procédé au phosphoramidite.

15. Utilisation des composés selon la revendication 14, pour le marquage d'oligonucléotides en position 5'.
